# EUROPEAN PATENT SPECIFICATION

(11) **EP 4 306 067 B1**
(45) Date of publication and mention of the grant of the patent: **02.12.2026**
(21) Application number: 23182744.5
(22) Date of filing: 30.06.2023
(51) Int. Cl.: A61B 17/80

(54) **BONE PLATE FOR FIXING A BONE FRAGMENT TO A BONE**
KNOCHENPLATTE ZUR FIXIERUNG EINES KNOCHENFRAGMENTS AN EINEM KNOCHEN
PLAQUE OSSEUSE DE FIXATION D'UN FRAGMENT OSSEUX À UN OS

(30) Priority: 13.07.2022 US 202217863796
(43) Date of publication of application: 17.01.2024
(73) Proprietor: Stryker European Operations Limited, Carrigtwohill, Co. Cork T45 HX08 (IE)
(72) Inventor: KEIL, Isabel, 79111 Freiburg (DE); SYLVESTRE, Pierre-Luc, CH-2540 Grenchen (CH); BARLOW, John, Rochester, MN, 55905 (US)
(74) Representative: Röthinger, Rainer

(56) References cited:
- US-A1- 2005 240 187
- US-A1- 2012 179 208
- US-A1- 2016 317 205
- US-A1- 2016 324 552
- US-A1- 2021 186 579

## Description

### Technical Field

The present disclosure generally relates to bone plates. In particular, a bone plate for fixing a bone fragment to a bone is disclosed which comprises positioning means for an initial bone plate fixation and position adjustment. The bone plate may be applied for fragmented long bones, in particular for fragmented bone of the proximal humerus.

### Background

To promote the healing process of a damaged or fragmented bone, bone plates are commonly attached to an outside surface of the bone. The bone plates act as stabilizing elements for the damaged area. In addition, they may help to position fragmented bone sections relative to one another.

For attaching a bone plate to a bone, various fixation techniques have been suggested. For example, screws may be applied which are inserted in through-holes of the bone plate and then screwed into an adjacent bone portion. Likewise, circumferential wires can be wrapped around a cross-section of the bone and a bone plate arranged at the bone. The wires are then tightened to secure the bone plate at the bone and to avoid a relative movement therebetween.

For a successful healing process of the damaged or fragmented bone without any subsequent movement restrictions and/or pain, it is crucial that the bone plate is correctly positioned relative to the fragmented bone. To that end, it is known to provide an oblong screw hole in the bone plate, which allows for a vertical plate adjustment using a screw. Additionally, a K-Wire may be inserted into a K-Wire opening for verifying a correct position of the bone plate by X-ray imaging. If the initially chosen position is so inaccurate that it cannot be adjusted by unscrewing the screw for releasing compression of the plate to the bone and gliding the screw in and along the oblong screw hole of the bone plate, the screw might need to be completely removed from the bone and re-placed at another, corrected position, thereby leaving a relatively large opening in the bone. Especially when treating proximal humerus fractures, one of the main surgical goals is finding the correct position of the bone plate.

From US 2013/0053899 A1, a bone plate with a hook portion having an elongated guiding opening is known. The elongated guiding opening is configured to receive a K-Wire along which the hook portion may be slid for guiding the bone plate towards a bone fragment. The height of the plate relative to the bone to which the plate is to be attached is defined by a bent portion of the hook portion configured to grasp a bone fragment.

From US 8,936,615 B2, a bone plate with two elongated holes is known which each are configured to receive a K-Wire inserted into a respective bone segment separated by a bone gap. By applying a force to the K-Wires, at least one of the bone segments is translated with respect to the other bone segment so that the bone gap is reduced.

From US 2016/317205 A1, a bone plate having a head portion with an elongated aperture is known which is configured to receive a K-wire. US 2005/240187 A1 discloses a bone plate with a head portion having two elongate suture openings adjacent the perimeter of the bone plate and adjacent to a pluarlity of fastening openings fir receiving a respective bone screw. US 2021/186579 A1 discloses a bone plate having a head portion with a slot used for guiding a screw through the plate and into an implant.

### Summary

There is a need for a bone plate which can be reliably positioned relative to the bone fragment while minimizing the damage caused to the bone.

The present invention relates to a bone plate as claimed hereafter. Preferred embodiments are set forth in the dependent claims. The bone plate comprises, inter alia, a proximal bone plate portion including a proximal longitudinal end of the bone plate and a distal bone plate portion including a distal longitudinal end of the bone plate, wherein the bone plate extends from the proximal longitudinal end to the distal longitudinal end in a longitudinal direction. The proximal bone plate portion is configured to abut on the bone fragment and the distal bone plate portion is configured to abut on the bone. The proximal bone plate portion comprises a slotted positioning opening configured to receive an elongated rigid positioning member and two fastening openings each configured to receive a respective fastening screw for fastening the bone plate to at least one of the bone and the bone fragment. The slotted positioning opening has a length extension in the longitudinal direction that is longer than a width extension in a lateral direction perpendicular to the longitudinal direction. The two fastening openings are positioned in a lateral direction relative to the slotted positioning opening on respective different sides of the slotted positioning opening.

The bone plate may be used for every kind of bone, in particular for fixing one or more bone fragments to a bone. The bone may be a long bone, for example a humerus or a femur. The bone plate may be laterally placed on the bone. The bone fragment may be proximally located at the bone. The proximal bone plate portion may be located more proximal than the distal bone plate portion when attached to the bone. The proximal longitudinal end may be the outer longitudinal edge of the proximal bone plate portion and the distal longitudinal end may be the outer longitudinal edge of the distal bone plate portion. A distance between the outermost point of the proximal longitudinal end and the outermost point of the distal longitudinal end may define the length extension of the bone plate, i.e. the extension of the bone plate in a longitudinal direction.

In the case of a proximal humeral bone plate, for example, the proximal bone plate portion may be configured to abut on an epiphyseal region of the bone, in particular partly on an epiphyseal region and/or on a part of the epiphyseal region. The proximal bone plate portion may be also configured to abut on a metaphyseal region of the bone. The distal bone plate portion may be configured to abut on a diaphyseal region of the bone and/or on a metaphyseal region of the bone.

The proximal bone plate portion and/or the distal bone plate portion may be anatomically pre-formed so as to conform to the outer contour of the respective region. For example, they may be pre-bent so as to conform to the outer contour of the respective region. For example, the proximal bone plate portion may be anatomically pre-formed so as to conform to a convex surface of the bone in the epiphyseal region. For that purpose, the proximal bone plate portion, when viewing the bone plate from a side, may laterally project from the distal bone plate portion.

The slotted positioning opening may be configured such that a common screw used for fastening the bone plate to the bone cannot be inserted into the slotted positioning opening. In particular, the lateral dimension of the slotted positioning opening may be too small for receiving a common fastening screw. The slotted positioning opening may have a uniform width extension over its whole length extension (with the exception of the tip portion of the slotted positioning opening, which may have a rounded shape). The slotted positioning opening may have a slot-like shape, i.e. may form a narrow elongated opening. The slotted positioning opening may be non-threaded. The slotted positioning opening may be configured to receive an elongated positioning member other than a screw. The slotted positioning opening and the elongated positioning member are configured such that the elongated positioning member may slide along and in the slotted positioning opening. The elongated positioning member is a rigid elongated positioning member. The elongated positioning member may be non-threaded along its longitudinal extension, with the possibly exception of a threaded tip. The elongated positioning member may be a K-Wire or a bone pin. The slotted positioning opening and the elongated positioning member may be means for the initial bone plate fixation.

The slotted positioning opening has a length extension substantially parallel to the longitudinal direction of the bone plate. In other implementations not forming part of the present invention, the length extension of the slotted positioning opening is inclined with respect to the longitudinal direction, for example at an angle between 5° to 30°.

The slotted positioning opening is formed in the proximal bone plate portion and thus may be located at a proximal portion of the bone. The slotted positioning opening may, for example, be positioned on an epiphyseal region of the bone and/or a metaphyseal region of the bone. The slotted positioning opening may be positioned close to a calcar region of the bone. The elongated positioning member, when inserted in the slotted positioning opening and the underneath bone, may help to position the bone plate relative to the calcar region of the bone. The bone plate may comprise exactly one slotted positioning opening. The bone plate may also comprise two or more slotted positioning openings. The elongated positioning member may be adapted to both initially fix the bone plate to the bone and verify whether the bone plate is correctly positioned. The elongated positioning opening may protrude from the bone plate with an end when being inserted into the bone with the other end.

The two fastening opening provided laterally to the slotted positioning opening are located on both sides thereof. The two fastening openings are locking openings, in particular angularly stable locking openings. A locking mechanism may be provided in the periphery of the opening itself, or may be provided by means of a locking insert inserted into the opening. The locking mechanism may be a polyaxial or monoaxial locking mechanism. A fastening opening may have a circular form. A fastening opening may also have an oblong form.

The slotted positioning opening and the two fastening openings may be positioned on the bone plate so as to be positioned close to bone fragment of the bone when the bone plate is attached to the bone. The two fastening openings lateral to the slotted positioning opening may be positioned on the bone plate so as to be positioned on a metaphyseal region and/or on an epiphyseal region of the bone when the bone plate is attached to the bone.

The bone plate may be part of a bone plate system also comprising the elongated positioning member.

In some implementations, a maximum width extension of the slotted positioning opening may amount to approximately 1.0 to 3.0 mm, in particular to approximately 1.5 to 2.5 mm (e.g., to approximately 2.0 mm). In particular, the slotted positioning opening may be configured to receive an elongated positioning member with a maximum width extension of 2.0 mm, for example with a maximum diameter of 2.0 mm. Thus, the maximum width dimension of the slotted positioning opening may be approximately 2.0 mm, in particular slightly more than 2.0 mm. In each case, the slotted positioning opening has a width dimension that corresponds approximately to the width dimension of the elongated positioning member.

The slotted positioning opening may be configured to receive a K-Wire. As such, the elongated positioning member may be a K-Wire. The K-Wire has a smaller dimension than a common fastening screw used for fastening a bone plate to a bone. The K-Wire is adapted to both initially fix the bone plate to the bone and verify whether the bone plate is correctly positioned.

In some implementations, the distal bone plate portion may comprise an oblong opening configured to receive a fastening member and having a length extension in the longitudinal direction of the bone plate. Thus, the oblong opening may have a larger dimension in the longitudinal direction, i.e. a length, than in the lateral direction, i.e. a width. The oblong opening may function as an alternative positioning opening in case the bone quality is not sufficiently good on the region of the slotted positioning opening. The fastening member may be a screw, in particular a non-locking screw. As such, the oblong opening may be a screw opening. The non-locking screw may be a compression screw. The oblong portion may be provided in a shaft portion of the bone plate. The oblong opening may be so positioned in the bone plate that it is positioned on a diaphyseal region of the bone when the bone plate is attached to the bone.

In other implementations, a width extension of the slotted positioning opening, i.e. a width dimension, may be smaller than a width extension of the oblong opening, i.e. a width dimension.

In further implementations, the bone plate may comprise at least one of one or more further fastening openings positioned above the slotted positioning opening in the proximal bone plate portion closer to the proximal end than the slotted positioning opening, and further to two further fastening openings positioned directly adjacent and below the slotted positioning opening, one or more further fastening openings positioned below the slotted positioning opening closer to the distal end than the slotted positioning opening in at least one of the proximal bone plate portion and, optionally, in the distal bone plate portion. The two or more further fastening openings may be configured as described above with respect to the one or more fastening openings provided laterally to the slotted positioning opening. The two or more further fastening openings may be provided for fixing the proximal bone plate portion to the bone and/or one or more bone fragments. One or more of the further fastening openings may be configured to connect a targeting device for inserting the fastening members into the bone plate and bone to the bone plate. In particular, one or more of the further fastening openings may be configured to receive a respective targeting arm. These one or more further fastening openings may be monoaxial locking openings.

In some implementations, the two or more further fastening openings positioned below the slotted positioning opening are positioned between the slotted positioning opening and the oblong opening in the proximal bone plate portion and, optionally, in the distal bone plate portion, and, optionally, below the oblong opening closer to the distal end than the oblong opening in the proximal bone plate portion. Thus, a plurality of fastening openings and further fastening openings may be provided along the overall length extension of the bone plate.

In some configurations, the distal bone plate portion may be configured as not to comprise any fastening openings positioned in the lateral direction relative to the oblong opening and along the length extension of the oblong opening. Other than laterally to the slotted positioning opening where one or more fastening openings are formed, no fastening opening may be formed lateral to the oblong opening and along a length dimension of the oblong opening.

In some implementations, at least some of the further fastening openings further to the two further fastening openings positioned directly adjacent and below the slotted positioning opening may be locking openings. In particular, as described with respect to the fastening openings arranged laterally to the slotted positioning opening, at least some of the further fastening openings may be angularly stable locking openings.

In still further implementations, the proximal bone plate portion may comprise at least one of one or more K-Wire openings configured to receive a K-Wire and positioned above the slotted positioning opening in the proximal bone plate portion closer to the proximal end than the slotted positioning opening, and one or more K-Wire openings configured to receive a K-Wire and positioned below the slotted positioning opening closer to the distal end than the slotted positioning opening. The K-Wire openings may have a circular diameter. A K-Wire opening may have a smaller dimension, in particular a smaller diameter, than a fastening opening. The K-Wire holes may by non-threaded. The one or more K-Wire holes may be also configured to receive suture for stabilizing the bone plate/bone system. In the proximal bone plate portion, the K-Wire holes may be distributed at a peripheral edge zone of the proximal bone plate portion.

In some implementations, one or more of the one or more K-Wire openings, in particular two of the one or more K-Wire openings, may be configured to receive a pin of a targeting device adapted to insert the fastening members into the bone plate for preventing a rotation of bone plate relative to the targeting device.

In specific implementations, the bone plate may be a proximal humeral bone plate, the proximal bone plate portion may be configured to abut on a humeral portion in a region of a greater tuberosity, and the distal bone plate portion may be configured to abut on a humeral shaft portion. Specifically, the bone plate may be a lateral proximal humeral bone plate for treating fragmented bone at a proximal end of the humerus. In the case of a proximal humeral bone plate, the slotted positioning opening may be positioned so that an elongated positioning member (such as a K-Wire) inserted in the slotted positioning opening extends in or close to a calcar region of the humerus. The elongated positioning member may act as an indicator for plate position in regards of the calcar region.

The proximal bone plate portion has a width extension that is greater than the width extension of the distal bone plate portion. The proximal bone plate portion may provide a bone plate head and the distal bone plate portion may provide a bone plate shaft. The width extension of the proximal bone plate portion may tend to decrease with decreasing distance to the distal bone plate portion.

The proximal bone plate portion and the distal bone plate portion may adjoin each other. Thus, the proximal and distal bone plate portion may directly adjoin each other without an intermediate bone plate portion located therein between.

According to a further aspect not forming part of the present invention, a proximal humeral bone plate is provided which comprises a proximal bone plate portion including a proximal longitudinal end of the bone plate and a distal bone plate portion including a distal longitudinal end of the bone plate, wherein the bone plate extends from the proximal longitudinal end to the distal longitudinal end in a longitudinal direction. The proximal bone plate portion is configured to abut on a humeral portion in a region of a greater tuberosity and the distal bone plate portion is configured to abut on a humeral shaft portion. The proximal bone plate portion comprises a slotted positioning opening configured to receive a K-wire for extending in or close to a calcar region of the humerus, wherein the slotted positioning opening has a length extension in the longitudinal direction that is longer than a width extension in a lateral direction perpendicular to the longitudinal direction.

In some implementations of the further aspect, a maximum width extension of the slotted positioning opening may amount to approximately 1.0 to 3.0 mm, in particular to approximately 1.5 to 2.5 mm (such as to approximately 2.0 mm).

In still other implementations of the further aspect, the bone plate may comprise one or more fastening openings configured to receive a respective fastening element for fastening the bone plate to the humerus, wherein the one or more fastening openings are positioned in the lateral direction relative to the slotted positioning opening and along the length extension of the slotted positioning opening.

In some implementations, the distal bone plate portion may comprise an oblong opening configured to receive a fastening member and having a length extension in the longitudinal direction of the bone plate. The width extension of the slotted positioning opening may be smaller than a width extension of the oblong opening.

In other implementions, the proximal humeral bone plate may comprise at least one of one or more further fastening openings positioned above the slotted positioning opening in the proximal bone plate portion closer to the proximal longitudinal end than the slotted positioning opening, and one or more further fastening openings positioned below the slotted positioning opening closer to the distal longitudinal end than the slotted positioning opening in at least one of the proximal bone plate portion and the distal bone plate portion. In particular, the one or more further fastening openings positioned below the slotted positioning opening, if present, may be positioned at at least one of the positions between the slotted positioning opening and the oblong opening in at least one of the distal bone plate portion and proximal bone plate portion, and below the oblong opening closer to the distal longitudinal end than the oblong opening in the proximal bone plate portion.

In implementations where the proximal humeral bone plate comprises an oblong opening, the bone plate may be configured so that the distal bone plate portion may not comprise any fastening openings positioned in the lateral direction relative to the oblong opening and along the oblong opening.

In some implementations, at least some of the further fastening openings may be locking openings.

In some further implementations, the proximal bone plate portion may comprise at least one of one or more K-Wire openings configured to receive a K-Wire and positioned above the slotted positioning opening in the proximal bone plate portion closer to the proximal longitudinal end than the slotted positioning opening, and one or more K-Wire openings configured to receive a K-Wire and positioned below the slotted positioning opening closer to the distal longitudinal end than the slotted positioning opening. Specifically, the one or more of the one or more K-Wire openings, in particular one of the one or more K-Wire openings, may be configured to receive a pin of a targeting device adapted to insert the fastening members into the bone plate for preventing a rotation of bone plate relativ to the targeting device.

In further implementations, the proximal bone plate portion may have a width extension that is greater than the width extension of the distal bone plate portion.

In a still further implementation, the proximal bone plate portion and the distal bone plate portion may adjoin each other.

### Brief Description of the Drawings

Further details, advantages and aspects of the present disclosure will become apparent from the following implementations of the present disclosure taken in conjunction with the drawings, wherein:
- Fig. 1: schematically shows, in a front view, a proximal humeral bone plate according to an implementation of the present disclosure;
- Figs. 2a and 2b: schematically show the bone plate of Fig. 1 attached to proximal humerus, in a front view and a side view;
- Fig. 3: schematically shows a fluoroscopy image of the bone plate attached to the proximal humerus in Fig. 2b;
- Figs. 4a and 4b: schematically show the bone plate of Figs. 2a and 2b, after having adjusted the height position of the plate by gliding the bone plate along the K-Wire in a longitudinal direction, in a front view and a side view;
- Figs. 5a and 5b: schematically show a targeting device connected to a bone plate as an useful example for understanding some aspects of the present disclosure;
- Fig. 6: schematically shows, in a top view, a proximal humeral bone plate according to another implementation of the present disclosure;
- Fig. 7: schemtically shows a first cross-sectional view of the proximal humeral bone plate of Fig. 6;
- Fig. 8: schematically shows a second cross-sectional view of the proximal humeral bone plate of Fig. 6;
- Fig. 9: shows a method not forming part of the present invention but representing background art that is useful for understanding the present invention, the method being for adjusting a position of a humeral bone plate according to an implementation of the present disclosure, and
- Fig. 10: schematically shows a proximal bone plate portion of a proximal humeral bone plate according to the present disclosure with screws inserted into the proximal bone plate portion and the proximal humerus.

### Detailed Description

In the following, an implementation of a bone plate according to the present disclosure will be described.

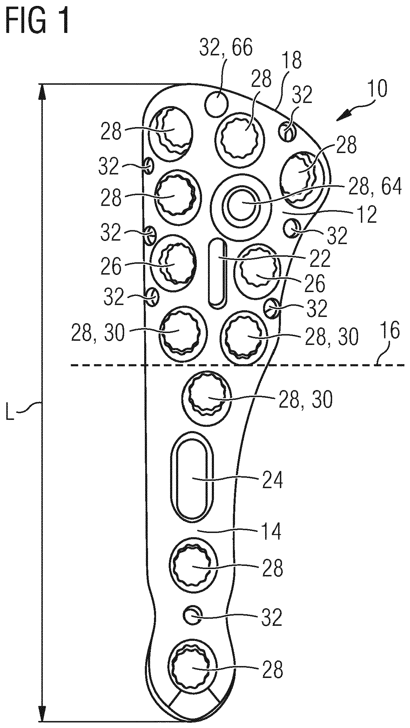

The bone plate 10 illustrated in Fig. 1 is, to exemplify the present disclosure, a proximal humeral bone plate. The proximal humeral bone plate is designed to treat fractures of the proximal humerus. To that end, the bone plate comprises a plurality of openings for adjusting and/or attaching the bone plate relative to the bone including the bone fragment. The attachment may encompass one or both of a temporary attachment and a long-term attachment.

In detail, the bone plate 10 comprises a proximal bone plate portion 12 and a distal bone plate portion 14 which are directly adjacent to each other, i.e. adjoin each other. A dashed line 16 is inserted in Fig. 1 dividing the bone plate 10 into the proximal bone plate portion 12 and distal bone plate portion 14, as chosen herein to exemplify the present disclosure. The proximal bone plate portion 12 comprises a proximal longitudinal end 18 of the bone plate 10 and the distal bone plate portion 14 comprises a distal longitudinal end 20 of the bone plate 10. The proximal longitudinal end 18 is defined by a proximal peripheral edge of the bone plate 10 and the distal longitudinal end 20 is defined by a distal peripheral edge of the bone plate 10. A distance between the most proximal and most distal points at the proximal and longitudinal ends 18, 20 defines a length L (length extension in a longitudinal direction) of the bone plate 10. The proximal bone plate portion has a width extension perpendicular to the length extension that is greater than the width extension of the distal bone plate portion. In particular, the bone plate 10 has a width extension that tends to decrease from the proximal longitudinal end to the distal longitudinal end. The greatest width extension is smaller than the length extension L of the bone plate 10. The proximal bone plate portion 12 corresponds to a bone plate head and the distal bone plate portion 14 corresponds to a bone plate shaft.

The proximal bone plate portion 12 is intended to cooperate with a bone fragment or bone fragments. In particular, the proximal bone plate portion 12 is intended to contact the bone fragment or bone fragments. More particularly, the proximal bone plate portion 12 is configured to abut on a humeral portion in a region of a greater tuberosity. For that purpose, the proximal bone plate portion 12 may be anatomically pre-formed so as to conform to a convex surface of the humerus in a region of the greater tubercle. The distal bone plate portion 14 is configured to contact a humeral shaft portion, which may be separated from the region of the greater tuberosity by one or more fracture lines. In some implementations, one or more fracture lines exist between the region of the greater tuberosity and the shaft portion. Thus, the one or more fracture lines may be formed in the shaft or in a region between the shaft and greater tuberosity, i.e. in the metaphyseal region. In some implementations, one or more fracture lines are formed additionally or alternatively in the epiphyseal region, for example in a region in or close to the greater tuberosity.

The proximal bone plate portion 12 comprises a slotted positioning opening 22 configured to receive an elongated positioning member. While in the present implementation described in the following with reference to the figures the positioning member is a K-Wire, in other implementations the positioning member may be any kind of rigid elongated member configured to be inserted in the slotted positioning opening 22 for preliminarily fixing and adjusting the bone plate 10, for example a pin or a wire in general. The slotted positioning opening 22 extends along a longitudinal direction of the bone plate 10 and has a larger dimension in the longitudinal direction than in a lateral direction perpendicular to the longitudinal direction. The longitudinal direction is the direction along the height (length extension) of the bone plate 10. In the present implementation, the slotted positioning opening 22 is configured to receive a K-Wire with a maximum diameter of 2.0 mm. Thus, the maximum dimension of the slotted positioning opening in the lateral direction, i.e. a maximum width dimension of the slotted positioning opening, is approximately 2.0 mm, in particular slightly larger than 2.0 mm, for example 2.05 mm or 2.5 mm. The dimension of the slotted positioning opening in the longitudinal direction, i.e. a length dimension of the slotted positioning opening, is approximately 1 cm. In the present implementation, the slotted positioning opening 22 is non-threaded and has a smooth and non-structured peripheral surface. In other implementations, the slotted positioning opening 22 may have a thread or another non-locking structure which allows, in cooperation with the associated positioning member, a longitudinal displacement of the bone plate 10 along the positioning member in the slotted positioning opening 22.

In other indications than for the proximal humerus, the dimensions of the slotted positioning opening may be different than described above. For example, in case of a femural bone plate, the slotted positioning opening may have a larger length dimension than described above.

The bone plate 10 of Fig. 1 further comprises an oblong opening 24 in the distal bone plate portion 14 that has a length extension in the longitudinal direction of the bone plate 10. The oblong opening 24 has a width dimension that is larger than the width dimension of the slotted positioning opening 22. The oblong opening 24 is configured and dimensioned to receive a fastening member, in particular a screw, more particularly a non-locking screw. Typical width dimensions of the oblong opening 24 are between 3 mm and 8 mm. Typical length dimensions of the oblong opening 24 are 8 mm to 15 mm.

In the present implementation, the oblong opening 24 functions as an alternative positioning opening, as will be explained below. Further in the present implementation, the oblong opening 24 has a multi-facetted structure at its periphery that is intended to cooperate with the screw head so as to exert compression forces in the bone segments in a direction corresponding to the longitudinal direction of the plate 10. The multi-facetted structure is configured such that it allows a displacement of the screw along the oblong opening 24. In other implementations, the oblong opening 24 may be non-threaded or may have a thread or another non-locking structure at its periphery. The thread or other non-locking structure may also allow, in cooperation with the associated fastening member, a longitudinal displacement of the bone plate 10 along the fastening member in the oblong opening 24.

In addition to the slotted positioning opening 22 and the oblong opening 24, the bone plate 10 of Fig. 1 comprises a plurality of fastening openings.

In the proximal bone plate portion 12, laterally to the slotted positioning opening 22 and along the length extension of the slotted positioning opening 22, one or more fastening openings 26 are formed. In the bone plate of Fig. 1, two fastening openings 26 are formed, a first one 26 on a with respect to Fig. 1 left side of the slotted positioning opening 22 and a second one 26 on a with respect to Fig. 1 right side of the slotted positioning opening 22.

Further fastening openings 28 are formed between the proximal end of the slotted positioning opening 22 and the proximal longitudinal end 18 of the bone plate 10, with respect to Fig. 1 above the slotted positioning opening 22. In particular, as is seen in Fig. 1, five further fastening openings 28 are formed in this region of the plate 10.

Also in the bone plate 10 of Fig. 1, five further fastening openings 28 are formed between the distal longitudinal end 20 of the bone plate 10 and distal end of the slotted positioning opening 22, wherein three of the five further fastening openings 28 are provided between the distal end of the slotted positioning opening 22 and the proximal end of the oblong opening 24 , and two of the five further fastening openings 28 are provided between the distal end of the oblong opening 24 and the distal longitudinal end 20 of the bone plate 10.

Thus, in the bone plate 10 of Fig. 1, fastening openings 26, 28 are provided in the proximal bone plate portion 12 laterally and along the slotted positioning opening 22, in a proximal direction with respect to the proximal end of the slotted positioning opening 22 and in a distal direction with respect to the distal end of the slotted positioning opening 22.

Generally, in the implementations according to the present disclosure, one or more of the fastening openings 26, 28 may be a locking opening. The one or more locking openings comprise a locking structure for locking a bone fastener received through that opening 26, 28 at a selected angle to the bone plate 10. The locking structure can include a threaded portion or a circumferential lip (or other structure) adapted to lockingly engage the bone fastener, in particular a threaded head thereof. In one variant, the locking structure has one or more protrusions extending in a radial direction towards the centre of the fastening opening 26, 28.

A bone fastening element can be polyaxially or monoaxially insertable through the fastening opening 26, 28, such that the locking structure may receive a head portion of a fastening element for locking engagement. A fastening element may have a self-cutting portion that can be inserted into the fastening opening 26, 28 for engaging the locking structure.

In the implementation illustrated in Fig. 1, the fastening openings 26, 28 of the bone plate 10 are locking openings, in particular angularly stable locking openings. At least one of the fastening openings 26, 28 has a monoaxial locking structure and is adapted to cooperate with a targeting device, as will be explained in detail. The fastening openings 26, 28 other than the one or more fastening openings having a monoaxial locking structure have a polyaxial locking structure. Furthermore, in the implementation shown in Fig. 1, the fastening openings 26, 28 have a circular shape.

Multiple fastening openings 30 of the fastening openings 28 are intended for receiving calcar screws, which, with the bone plate 10 correctly positioned on the proximal humerus, are positioned with their tips in the calcar region when being inserted into the bone. In the bone plate of Fig. 1, three fastening openings 30 for the calcar screws, i.e. calcar openings 30, are provided between the slotted positioning opening 22 and the oblong opening 24. The slotted positioning opening 22 is located next to the fastening openings 30 for the calcar screws, which is an important aspect of the present disclosure as outlined in more detail below.

Further to the above-described openings, the bone plate 10 of Fig. 1 comprises circular K-Wire openings 32 configured to receive a K-Wire for aligning the bone plate 10 and/or preliminarily fixing the bone plate 10 with respect to the bone. In the implementation of Fig. 1, a plurality of K-Wire openings 32 is provided at a peripheral edge region of the proximal bone plate portion 12, i.e. along the whole contour of the proximal bone plate portion 12, and one K-Wire opening 32 is provided close to the distal longitudinal end 20 of the distal bone plate portion 14 between two further fastening openings 28.

In the implementation of Fig. 1, the K-Wire openings 32 are non-threaded. The K-Wire openings 32 may be also configured to receive fasteners, such as screws, for permanently fixing the bone plate 10 to the bone segments or to receive suture to enhance the stability of the bone plate - bone system. In particular, in the present implementation, at least some of the K-Wire openings 32 have a cross-sectional contour in a thickness direction of the bone plate 10 that is not cylindrical, but arch-shaped (i.e., curved), thereby being configured to receive a bent needle for threading a suture. More particularly, the respective K-Wire opening 32 is surrounded by a peripheral wall formed by the bone plate 10 that has a bent, in particular arch-shaped, contour in a thickness direction of the bone plate 10 so that the opposed needle input/output portions of the K-Wire opening 32 is closer to the outer rim of the bone plate 10 than a central portion of the K-Wire opening 32 located between the needle input/output portions.

As will be described below, at least one of the K-Wire openings 32 is also configured to cooperate with a targeting device so as to prevent a rotation of the bone plate 10 against the targeting device.

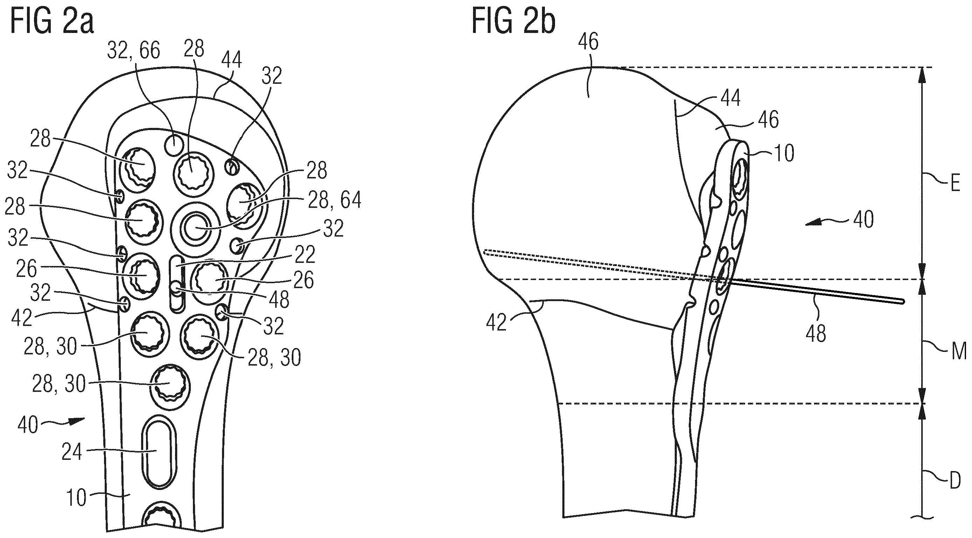

By means of the following Figs. 2a to 4b and Fig. 9, a method for (initially) positioning and fixing the bone plate 10 of Fig. 1 to a fragmented humerus is shown. The method does not form part of the present invention and represents background art that us useful for understanding the invention.

The humerus 40 shown in Figs. 2a and 2b comprises fractures at two positions, as is schematically illustrated by lines 42, 44. Line 44 denotes a fracture in a region of the greater tuberosity of the humerus 40, and line 42 denotes a fracture in a transition region between the humerus shaft S (diaphyseal region D) and the greater tuberosity. The greater tuberosity is part of the epiphyseal region E of the humerus 40, and the transition region is the metaphyseal region M of the humerus 40. Thus, the illustrated humerus 40 comprises three bone fragments 46 at a proximal region of the humerus 40, and the bone plate 10 is a proximal humeral bone plate, in particular a proximal lateral humeral bone plate.

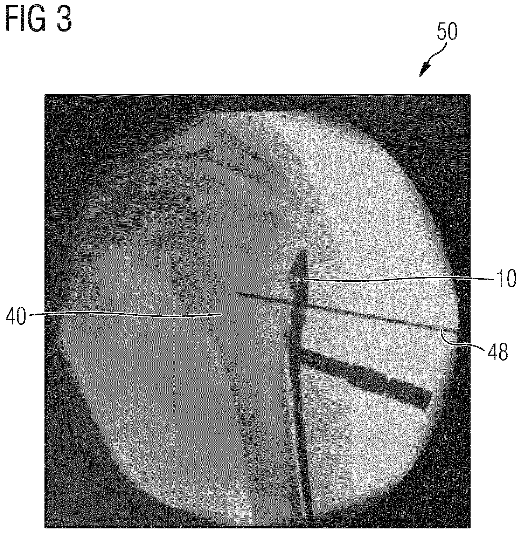

As is illustrated in Figs. 2, in a first step, the bone plate 10 is provisionally aligned with the bone. This first step corresponds to the step 300 of Fig. 3 where a bone plate is positioned on a humerus. For that purpose, the bone plate 10 is placed on the humerus 40 so as to extend over the two bone fragments 46 in the epiphyseal region E, the metaphyseal region M and a shaft portion in the diaphyseal region D directly adjoining the metaphyseal region M. In particular, the bone plate 10 is positioned so as to be centred against the lateral aspect of the greater tuberosity. Furthermore, the proximal longitudinal end 18 of the bone plate 10 is placed a predetermined distance below the superior aspect of the greater tuberosity. A typical predetermined distance is approximately 10 mm, for example 10.5 mm, 11 mm or 9.5 mm.

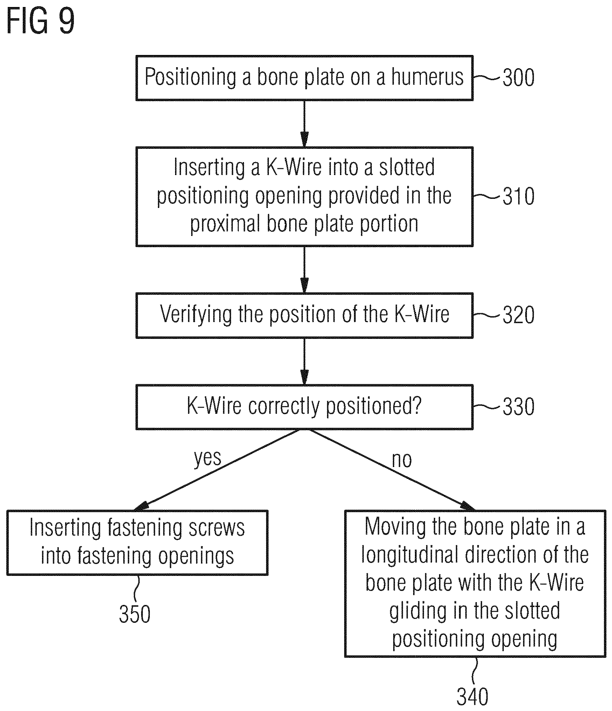

Then, in a next step, a K-Wire 48 is inserted in the slotted positioning opening 22 and into the bone next to the slotted positioning opening 22. Advantageously, the K-Wire 48 is inserted so as to be positioned in or close to a middle position of the slotted positioning opening 22. This step corresponds to step 310 of Fig. 9, where a K-Wire is inserted into a slotted positioning opening provided in the proximal bone plate portion.

Figs. 2a and 2b show the bone plate 10 provisionally aligned as described above with the K-Wire 48 inserted into the slotted positioning opening 22 and into the bone underneath.

In a subsequent step, as is illustrated in Fig. 3, an X-ray picture 50 is taken by fluoroscopy from the proximal humerus 40 and the provisionally aligned bone plate 10 with the inserted K-Wire 48, showing the bone plate 10 in a side view, for verifying whether the bone plate 10 is correctly positioned on the proximal humerus 40, and in particular with respect to the calcar region of the proximal humerus 40. Since the K-Wire 48 is located proximal to fastening openings 28, 30 intended to receive the calcar screws and thus ends up in or at least close to the calcar region, which is a region with good bone quality in terms of a thickness and density of the cortical bone, the K-Wire 48 enables an analysis of the longitudinal position of the bone plate 10 in the calcar region.

The calcar region is a designated area at the base of the humerus head on its medial aspect. The humeral calcar refers to the inferomedial cortical area where the humeral head extends to the surgical neck of the humerus. For example, in Fig. 2b, it is, at least roughly, the portion of the humerus at which the K-Wire 48 heads. In Fig. 3, it is approximately the region where the line associated with the reference numeral 40 ends.

Before taking an X-ray picture, the position of the bone plate 10 may be checked by visual inspection only. If the result of the visual inspection is that the bone plate is clearly not correctly positioned, the bone plate 10 may be also adjusted by displacing the bone plate 10 in a proximal or distal direction of the humerus 40 without before taking an X-ray picture. In other cases, i.e. if it is not obvious that the bone plate 10 is not correctly positioned and/or for additionally verifying the result of the visual inspection, an X-Ray picture is taken as described above after the visual inspection.

The above-described steps of the visual inspection and/or taking an X-ray picture are exemplified by step 320 in Fig. 9, where the position of the K-Wire is verified.

If it is concluded that the bone plate longitudinal (height) position is not correct, in a further subsequent step, the plate 10 is adjusted by displacing the bone plate 10 in a proximal or distal direction of the humerus 40, thereby gliding the K-Wire 48 in the slotted positioning opening 22. This is exemplified by steps 330 and 340 of Fig. 9, where, if it is concluded that the K-Wire is not correctly positioned, the bone plate is moved in a longitudinal direction of the bone plate with the K-Wire gliding in the slotted positioning opening.

In some cases, the provisional alignment may be so incorrect that it cannot be adjusted by moving the K-Wire 48 along the slotted positioning opening 22 a distance pre-defined by the longitudinal dimension of the slotted positioning opening 22. In these cases, the K-Wire 48 has to be removed and re-placed at a new, corrected position.

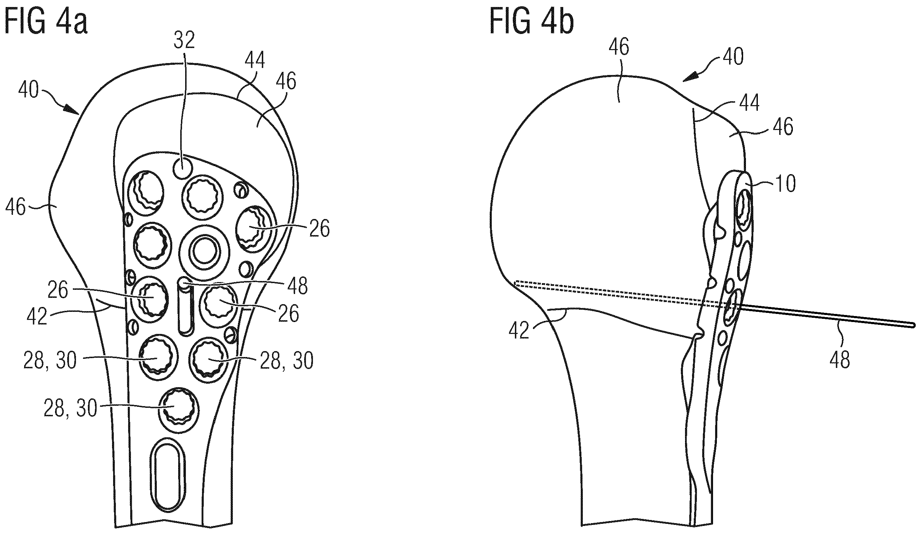

In Figs. 4a and 4b, the bone plate 10 is shown adjusted compared to the bone plate 10 shown in Figs. 2a and 2b. In particular, the bone plate 10 is shown to be shifted in a distal humeral direction.

The X-Ray imaging step described with reference to Fig. 3 and the plate height adjustment step described with reference to Figs. 2 and 4 is repeated until it is determined that the bone plate 10 is correctly positioned.

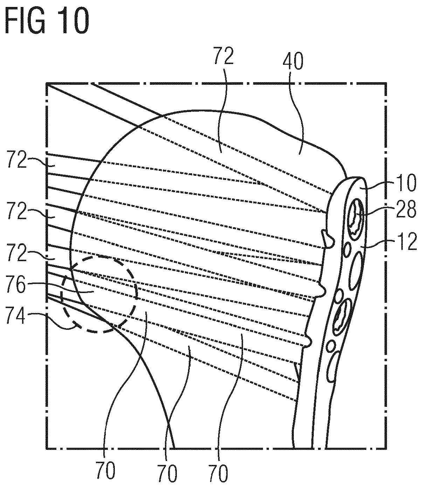

If it is determined that the bone plate position is correct, in a next step, calcar screws (designated with reference numeral 70 in Fig. 10 described below) are inserted in the fastening openings 28, 30 and into the bone. In this implementation of the method, the calcar screws are locking screws and the fastening openings 28, 30 have a polyaxial locking mechanism. In other implementations, the fastening openings 28, 30 may have a monoaxial locking mechanism or may be non-locking openings. Inserting the calcar screws is done in a manner commonly known. For example, prior to inserting the calcar screw manually, a drill sleeve is inserted in the respective fastening hole 28, 30.

These steps are exemplified by steps 330 and 350 of Fig. 9, where, it is concluded that the K-Wire is correctly positioned, fastening screws are inserted into fastening openings.

Subsequently, the position of the respective calcar screw is checked, for example by fluoroscopy. In case the position and/or orientation is not correct, the calcar screw is removed and re-placed at a new, corrected position. If, for correcting the position of the calcar screw the bone plate 10 has to be moved in a longitudinal direction a distance larger than allowed by the slotted positioning opening 22, also the K-Wire 48 is removed, the bone plate height is adjusted and the K-Wire 48 is re-inserted in the slotted positioning opening at the adjusted position before the calcar screw is inserted in the respective fastening opening 28, 30 and the position of the calcar screw is re-checked.

The steps described in the last paragraph are repeated until it is determined that the calcar screw position is correct.

After that, the other screws are placed in the fastening openings 26, 28 in the proximal bone plate portion 12, in a manner commonly known. For example, before inserting the other screws, the bone plate 10 may be preliminarily fixed by inserting K-Wires in one or more of the circular K-Wire openings 32 in the proximal bone plate portion 12.

Finally, screws are placed in the fastening openings 28 in the distal bone plate portion 14, also in a manner commonly known. The oblong opening 24 may be also used as a fastening opening by inserting a screw therein and in the underneath bone.

The oblong opening 24 in the distal bone plate portion 14 functions as an alternative positioning opening in case the bone quality is not sufficiently good in the bone area where the K-Wire 48 is used in combination with the slotted positioning opening 22. The oblong opening 24 is provided at the junction of the peri-articular area of the bone plate 10 and a shaft portion located at a distal end region of the distal bone plate portion 14. If the oblong opening 24 is used as the positioning opening, the bone plate 10 is placed on the bone portion concerned as described above, and instead of inserting the K-Wire 48 in the slotted positioning opening 22, a screw, in particular a non-locking screw, is inserted in the oblong opening 24 and the bone underneath for preliminary fixation of the bone plate 10. A K-Wire may be additionally inserted in the circular K-Wire opening 32 of the distal bone plate portion 14. Then, the position of the bone plate 10 is checked by fluoroscopy, for example, and if adjustment is required, the bone plate 10 may be moved in a longitudinal direction along the screw in the oblong opening 24 (after having removed the K-Wire, if present).

When it is verified that the position is correct, fastening screws are inserted in the distal bone plate portion 14, before fixation of the proximal bone plate portion 12 by inserting screws in the proximal bone plate portion 12 in a known manner.

Fig. 10 schematically illustrates a proximal bone plate portion 12 with calcar screws 70 inserted into the calcar openings 30 and other screws 72 inserted into the fastening openings 26, 28 and into the proximal humerus. The calcar region 74 is schematically designated by a broken line forming a circle. As is seen in this figure, the tips 76 of the calcar screws 70 go into and fixate to the calcar region 74 as that is the densest bone portion.

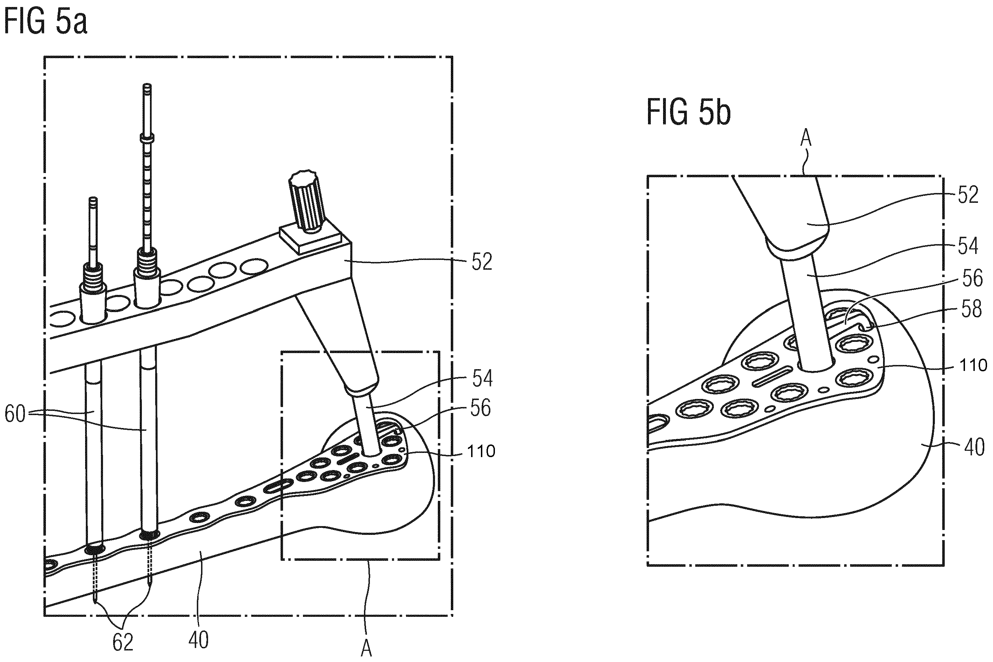

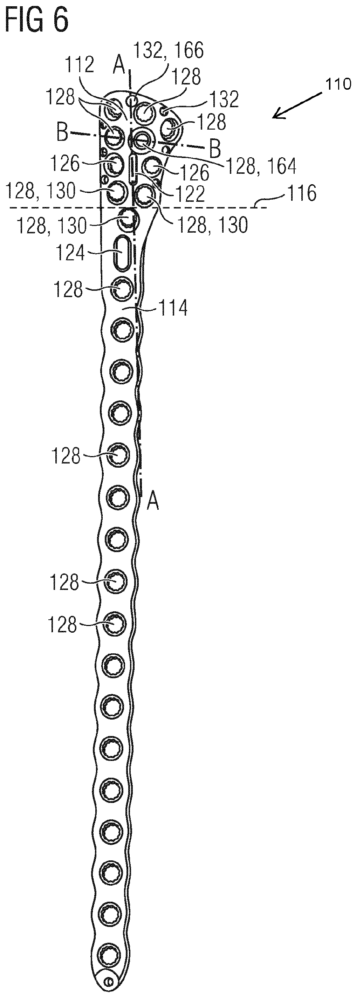

Figs. 5a and 5b show a targeting device 52 connected to a bone plate 110. The bone plate 110 is configured similarly as the bone plate 10 and will be described in detail below with respect to Figs. 6, 7 and 8. Fig. 5b is an enlarged view of the image section A of Fig. 5a.

As is seen in these figures, the targeting device 52 has a targeting arm 54 inserted into a fastening opening of the bone plate 110 for connecting the targeting device 52 to the bone plate 110. A pin 56 laterally projects from the targeting arm 54 which has a pin end 58 configured to be received by one of the openings in the bone plate 110. In this manner, a rotation of the plate against the targeting device 52 is prevented.

Further seen in Fig. 5a are drill sleeves 60 with corresponding drills 62 inserted into the humerus 40 by the targeting device 52.

Referring now again to Fig. 1 of the drawings, at least some of the fastening openings 26, 28 is configured so as to be also able to receive the targeting arm 54 of the targeting device 52. In the example of Fig. 1, the fastening opening 64 is configured to receive the targeting arm 54. The fastening opening 64 also has a monoaxial locking mechanism configured to receive a locking screw. The bone plate 110 has a corresponding fastening opening 164 configured to receive the targeting arm 54, as will be described below with reference to Figs. 6, 7 and 8.

Furthermore, in the bone plate 10 of Fig. 1, also at least one of the K-Wire openings 32 is configured to receive the pin 56, in particular the pin end 58 of the pin 56, to prevent a rotation of the bone plate 10 against the targeting device 52. In the example of Fig. 1, one of the K-Wire openings 32 adjacent to the proximal longitudinal end 18, additionally designated with reference numeral 66, is configured to receive the pin 56. The bone plate 110 has a corresponding K-Wire opening 166 configured to receive the pin 56, as will be described below.

The K-Wire opening 66 is designed to allow the pin 56 limiting the rotation of the targeting device 52, which can create enormous moments/forces. To that end, in the present implementation, the K-Wire opening 66 has a larger diameter than the K-Wire openings 32.

Fig. 6 shows another implementation of a proximal humeral bone pate. Same or corresponding features are designated with corresponding reference numerals, respectively increased by 100.

The proximal humeral bone plate 110 structurally corresponds to the proximal humeral bone plate 10 described with reference to Figs. 1 to 4, with the exception that the distal bone plate portion 114 has a longer length extension than the distal bone plate portion 14 of Figs. 1 to 4. Thus, the proximal humeral bone plate 110 comprises a proximal bone plate portion 112, a distal bone plate portion 114, a slotted positioning opening 122, an oblong opening 124, two fastening openings 126 laterally and along the slotted positioning opening 122, a plurality of further fastening openings 128, and K-Wire openings 132.

Since the distal bone plate portion 112, which corresponds to a shaft portion of the bone plate 110, has a longer length extension, more further fastening openings 128 are provided in the distal bone plate portion 114 compared to the number of further fastening openings 28 in the distal bone plate portion 14 of Fig. 1.

As in the implementation of Fig. 1, some 130, in particular three, of the further fastening openings 128 are calcar openings 130. Furthermore, one 164 of the further fastening openings 128 is configured to receive the targeting arm 54 of the targeting device 52, and one 166 of the K-Wire openings 132 is configured to receive the pin 56, in particular the pin end 58 of the pin 56, to prevent a rotation of the bone plate 10 against the targeting device 52.

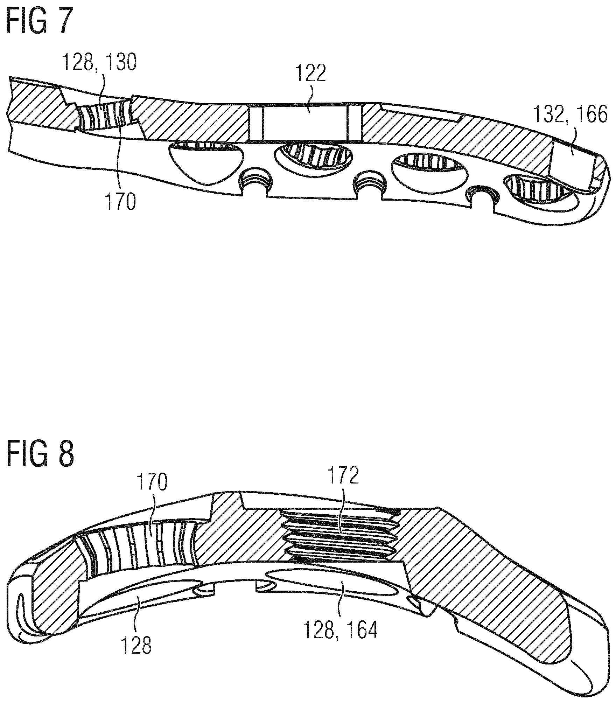

Fig. 7 shows a cross-section of the bone plate 110 of Fig. 6, in a thickness direction of the bone plate 110 and along the Line A in Fig. 6, and in an enlarged view. The cross-section goes though the calcar opening 132, 166, the slotted positioning opening 122 and one of the calcar openings 130. As is seen in this figure, in this implementation, the slotted positioning opening 122 is non-threaded and has a smooth, non-structured peripheral surface. Similarly, the K-Wire opening 132, 166 is non-threaded. The calcar opening 130 is surrounded by a peripheral wall having a surface with a polyaxial locking structure 170. The polyaxial locking structure 170 may be formed in the material of the bone plate 110, or may be provided by means of a locking insert inserted into an opening formed on the bone plate 110. The polyaxial locking structure 170 may be a polyaxial locking structure of an engagement region included in a bone plating system as shown in Figs. 57 to 72 of EP 3 533 403 A1 and described therein. Specifically, the polyaxial locking structure of Figs. 57 to 72 of this document comprises a plurality of spaced apart scalloped regions and screw-engaging members which are spaced apart about the central axis of the opening. When a locking screw is positioned within opening, threads engage screw-engaging members of the opening by plastically deforming the screw-engaging members to secure the locking screw to the bone plate at variable angles.

Fig. 8 shows another cross-section of the bone plate 110 of Fig. 6, in a thickness direction of the bone plate 110 and along the Line B in Fig. 6, and also in an enlarged view. The cross-section goes through two further fastening opening 128, wherein one 128, 164 of the two further fastening openings 128 is the further fastening opening 128 configured to receive the targeting device 52. As is seen in this figure, the further fastening opening 128 not configured to cooperate with the targeting device 52 has a peripheral surface with a polyaxial locking structure 170. The further fastening opening 128, 164 configure to receive the targeting arm 54 has a peripheral surface with a monoaxial locking structure 172.

As becomes clear from the above explanations, the position of a proximal humeral bone plate according to the present disclosure may be adjusted by the following (not claimed) steps schematically illustrated by Fig. 9:
- in a step 300, positioning the bone plate on a humerus so that a proximal bone plate portion abuts on a humeral portion in a region of a greater tuberosity and a distal bone plate portion abuts on a humeral shaft portion,
- in a step 310, inserting a K-Wire into a slotted positioning opening provided in the proximal bone plate portion so that the K-Wire extends into a calcar region of the humerus or is positioned close to the calcar region of the humerus,
- in steps 320, 330, verifying whether the K-Wire is correctly positioned in the calcar region or close to the calcar region,
- if it is determined that the K-Wire is not correctly positioned, in a step 340, moving the bone plate in a longitudinal direction of the bone plate with the K-Wire gliding in the slotted positioning opening for adjusting the bone plate position, and
if it is determined that the K-Wire is correctly positioned, in a step 350, inserting fastening screws into fastening openings in the proximal bone plate portion.

The step of verifying whether the K-Wire is correctly positioned may be a visual inspection by the surgeon. Optionally, the step of verifying the correct position may also comprise taking an image by an appropriate imaging technique, for example by taking an X-Ray image.

Furthermore, the step 350 of inserting fastening screws into fastening openings in the proximal bone plate portion comprises inserting one or more calcar screws into respective one or more fastening openings provided in the proximal bone plate portion so as to protrude into the humerus close to the calcar region or into the calcar region of the humerus.

The bone plate according to the present disclosure has a slotted positioning opening which has a relatively small width extension and thus can be placed in a proximal portion of the bone plate. The slotted positioning opening is configured to receive an elongated rigid positioning member, with relatively small dimensions. The elongated rigid positioning member is both adapted to initially fix the bone plate to the bone as well as to be a verifying member whether the bone plate is correctly positioned. No other members such as screws are needed. To adjust the position, the bone plate can be moved in a longitudinal direction with the elongated positioning member gliding in the slotted positioning opening.

The elongated rigid positioning member creates a relatively small footprint on the bone. Hence, the plate position can be changed easily, even of the position is completely wrong and the elongated positioning member has to be re-placed in the bone.

If the bone plate is a proximal humeral bone plate, the elongated rigid positioning member inserted into the slotted positioning opening is placed close or in the calcar region, so that the bone plate position with respect to the calcar region can be verified.

## Claims

1. A bone plate (10, 110) for fixing bone fragments (46) to a bone (40), comprising
a proximal bone plate portion (12, 112) including a proximal longitudinal end (18) of the bone plate (10, 110) and a distal bone plate portion (14) including a distal longitudinal end (20) of the bone plate (10, 110), the bone plate (10, 110) extending from the proximal longitudinal end (18) to the distal longitudinal end (20) in a longitudinal direction, and the proximal bone plate portion (12, 112) having a width extension that is greater than a width extension of the distal bone plate portion (14, 114),
the proximal bone plate portion (12, 112) being configured to abut on a bone fragment (46) and the distal bone plate portion (14, 114) being configured to abut on the bone (40),
the proximal bone plate portion (12, 112) comprising a slotted positioning opening (22, 122) having a length extension parallel to the longitudinal direction of the bone plate (10, 110) and configured to receive an elongated rigid positioning member (48) and two fastening openings (26, 126) each configured to receive a respective fastening screw (72) for fastening the bone plate (10, 110) to at least one of the bone (40) and the bone fragment (46),
wherein the length extension of the slotted positioning opening (22, 122) in the longitudinal direction of the bone plate (10, 110) is longer than a width extension of the slotted positioning opening (22, 122) in a lateral direction perpendicular to the longitudinal direction,
wherein the width extension of the slotted positioning opening (22, 122) substantially corresponds to a width dimension of the elongated rigid positioning member (48) and the slotted positioning opening (22, 122) is configured to allow the elongated rigid positioning member (48) to slide along and in the slotted positioning opening (22, 122),
**characterized in that**
the two fastening openings (26, 126) are positioned in the lateral direction relative to the slotted positioning opening (22, 122) on respective different sides of the slotted positioning opening (22, 122),
wherein the two fastening openings (26, 126) comprise a locking structure configured to lock the respective fastening element at a selected angle to the bone plate (10, 110), and
wherein the bone plate (10, 110) comprises two further fastening openings (28, 30, 128, 130) positioned directly adjacent and below the slotted positioning opening (22, 122) closer to the distal longitudinal end (20) than the slotted positioning opening (22, 122) in the proximal bone plate portion (12, 112), wherein the two further fastening openings (28, 30, 128, 130) comprise a locking structure configured to lock the respective fastening element at a selected angle to the bone plate (10, 110).

2. The bone plate (10, 110) of any of claim 1, wherein
a maximum width extension of the slotted positioning opening (22, 122) amounts to aprroximately 1.0 to 3.0 mm.

3. The bone plate (10, 110) of claim 1 or 2, wherein
the slotted positioning opening (22, 122) is configured to receive a K-Wire (48) as the elongated rigid positioning member.

4. The bone plate (10, 110) of any of the preceding claims, wherein
the distal bone plate portion (14, 114) comprises an oblong opening (24, 124) configured to receive a fastening member (72) and having a length extension in the longitudinal direction of the bone plate (10, 110).

5. The bone plate (10, 110) of claim 4, wherein
the width extension of the slotted positioning opening (22, 122) is smaller than a width extension of the oblong opening (24, 124).

6. The bone plate (10, 110) of any of the preceding claims, wherein
the bone plate (10, 110) comprises at least one of
- one or more further fastening openings (28, 64, 128, 164) positioned above the slotted positioning opening (22, 122) in the proximal bone plate portion (12, 112) closer to the proximal longitudinal end (18) than the slotted positioning opening (22, 122), and
- further to the two further fastening openings (28, 30, 128, 130) positioned directly adjacent and below the slotted positioning opening (22, 122), one or more further fastening openings (28, 30, 128, 130) positioned below the slotted positioning opening (22, 122) closer to the distal longitudinal end (20) than the slotted positioning opening (22, 122) in at least one of the proximal bone plate portion (12, 112) and the distal bone plate portion (14, 114).

7. The bone plate (10, 110) of claim 6, wherein the one or more further fastening openings (28, 30, 128, 130) positioned below the slotted positioning opening (22, 122) are positioned
- between the slotted positioning opening (22, 122) and the oblong opening (24, 124) in the proximal bone plate portion (12, 112) and, optionally, the distal bone plate portion (14, 114), and, optionally,
- below the oblong opening (24, 124) closer to the distal longitudinal end (20) than the oblong opening (24, 124) in the distal bone plate portion (14, 114).

8. The bone plate (10, 110) of any of claims 4 to 7, wherein
the distal bone plate portion (14, 114) does not comprise any fastening openings positioned in the lateral direction relative to the oblong opening (24, 124) and along the oblong opening (24, 124).

9. The bone plate (10, 110) of any of claims 6 to 8, wherein
at least some of the further fastening openings (28, 30, 64, 128, 130, 164) further to the two further fastening openings positioned directly adjacent and below the slotted positioning opening (28, 30, 128, 130) are locking openings.

10. The bone plate (10, 110) of any of the preceding claims, wherein
the proximal bone plate portion (12, 112) comprises at least one of
- one or more K-Wire openings (32, 66, 132, 166) configured to receive a K-Wire (48) and positioned above the slotted positioning opening (22, 122) in the proximal bone plate portion (12, 112) closer to the proximal longitudinal end (18) than the slotted positioning opening (22, 122), and
- one or more K-Wire openings (32, 132) configured to receive a K-Wire (48) and positioned below the slotted positioning opening (22, 122) closer to the distal longitudinal end (14) than the slotted positioning opening (22, 122).

11. The bone plate (10, 110) of claim 10, wherein
one (66, 166) or more of the one or more K-Wire openings (32, 66, 132, 166), in particular one (66, 166) of the one or more K-Wire openings (32, 66, 132, 166), are configured to receive a pin (56) of a targeting device (52) adapted to insert the fastening members into the bone plate for preventing a rotation of bone plate (10, 110) relativ to the targeting device (52).

12. The bone plate (10, 110) of any of the preceding claims, wherein
the bone plate (10, 110) is a proximal humeral bone plate,
the proximal bone plate portion (12, 112) is configured to abut on a humeral portion in a region of a greater tuberosity, and
the distal bone plate portion (14, 114) is configured to abut on a humeral shaft portion.

13. The bone plate (10, 110) of claim 12, wherein
the slotted positioning opening (22, 122) is positioned so as to be close to a calcar region (74) of the humerus (40), when the proxial humeral bone plate is fixed to a humeral bone.

14. The bone plate (10, 110) according to any of the preceding claims, wherein
the proximal bone plate portion (12, 112) and the distal bone plate portion (14, 114) adjoin each other.

## Patentansprüche

1. Knochenplatte (10, 110) zur Fixierung von Knochenfragmenten (46) an einem Knochen (40), umfassend
einen proximalen Knochenplattenabschnitt (12, 112) mit einem proximalen Längsende (18) der Knochenplatte (10, 110) und einem distalen Knochenplattenabschnitt (14) mit einem distalen Längsende (20) der Knochenplatte (10, 110), wobei sich die Knochenplatte (10, 110) von dem proximalen Längsende (18) zu dem distalen Längsende (20) in einer Längsrichtung erstreckt, und wobei der proximale Knochenplattenabschnitt (12, 112) eine Breitenerstreckung aufweist, die grösser ist als eine Breitenerstreckung des distalen Knochenplattenabschnitts (14, 114),
wobei der proximale Knochenplattenabschnitt (12, 112) dazu eingerichtet ist, an einem Knochenfragment (46) anzuliegen, und der distale Knochenplattenabschnitt (14, 114) dazu eingerichtet ist, an dem Knochen (40) anzuliegen,
wobei der proximale Knochenplattenabschnitt (12, 112) eine geschlitzte Positionierungsöffnung (22, 122) umfasst, die eine Längenerstreckung parallel zur Längsrichtung der Knochenplatte (10, 110) aufweist und dazu eingerichtet ist, ein längliches starres Positionierungselement (48) aufzunehmen, und zwei Befestigungsöffnungen (26, 126), die jeweils dazu eingerichtet sind, eine jeweilige Befestigungsschraube (72) zum Befestigen der Knochenplatte (10, 110) an dem Knochen (40) und/oder dem Knochenfragment (46) aufzunehmen,
wobei die Längenerstreckung der geschlitzten Positionierungsöffnung (22, 122) in Längsrichtung der Knochenplatte (10, 110) länger ist als eine Breitenerstreckung der geschlitzten Positionierungsöffnung (22, 122) in einer lateralen Richtung senkrecht zur Längsrichtung,
wobei die Breitenerstreckung der geschlitzten Positionierungsöffnung (22, 122) im Wesentlichen einer Breitenabmessung des länglichen starren Positionierungselements (48) entspricht und die geschlitzte Positionierungsöffnung (22, 122) dazu eingerichtet ist, zu ermöglichen, dass das längliche starre Positionierungselement (48) entlang und in der geschlitzten Positionierungsöffnung (22, 122) gleitet,
**dadurch gekennzeichnet, dass**
die beiden Befestigungsöffnungen (26, 126) in seitlicher Richtung relativ zu der geschlitzten Positionierungsöffnung (22, 122) auf jeweils unterschiedlichen Seiten der geschlitzten Positionierungsöffnung (22, 122) gelegen sind,
wobei die beiden Befestigungsöffnungen (26, 126) eine Verriegelungsstruktur umfassen, die dazu eingerichtet ist, das jeweilige Befestigungselement in einem ausgewählten Winkel relativ zu der Knochenplatte (10, 110) zu verriegeln, und
wobei die Knochenplatte (10, 110) zwei weitere Befestigungsöffnungen (28, 30, 128, 130) umfasst, die in dem proximalen Knochenplattenabschnitt (12, 112) unmittelbar benachbart und unterhalb der geschlitzten Positionierungsöffnung (22, 122) näher an dem distalen Längsende (20) gelegen sind als die geschlitzte Positionierungsöffnung (22, 122), wobei die zwei weiteren Befestigungsöffnungen (28, 30, 128, 130) eine Verriegelungsstruktur umfassen, die dazu eingerichtet ist, das jeweilige Befestigungselement in einem ausgewählten Winkel relativ zu der Knochenplatte (10, 110) zu verriegeln.

2. Knochenplatte (10, 110) nach Anspruch 1, wobei eine maximale Breitenerstreckung der geschlitzten Positionierungsöffnung (22, 122) ungefähr 1,0 bis 3,0 mm beträgt.

3. Knochenplatte (10, 110) nach Anspruch 1 oder 2, wobei die geschlitzte Positionierungsöffnung (22, 122) dazu eingerichtet ist, einen K-Draht (48) als das längliche starre Positionierungselement aufzunehmen.

4. Knochenplatte (10, 110) nach einem der vorhergehenden Ansprüche, wobei der distale Knochenplattenabschnitt (14, 114) eine längliche Öffnung (24, 124) aufweist, die zur Aufnahme eines Befestigungselements (72) ausgebildet ist und eine Längenerstreckung in Längsrichtung der Knochenplatte (10, 110) hat.

5. Knochenplatte (10, 110) nach Anspruch 4, wobei die Breitenerstreckung der geschlitzten Positionierungsöffnung (22, 122) kleiner ist als eine Breitenerstreckung der länglichen Öffnung (24, 124).

6. Knochenplatte (10, 110) nach einem der vorhergehenden Ansprüche, wobei die Knochenplatte (10, 110) aufweist
- eine oder mehrere weitere Befestigungsöffnungen (28, 64, 128, 164), die oberhalb der geschlitzten Positionierungsöffnung (22, 122) in dem proximalen Knochenplattenabschnitt (12, 112) näher an dem proximalen Längsende (18) als die geschlitzte Positionierungsöffnung (22, 122) gelegen sind, und/oder
- zusätzlich zu den zwei weiteren Befestigungsöffnungen (28, 30,128, 130), die unmittelbar benachbart und unterhalb der geschlitzten Positionierungsöffnung (22, 122) gelegen sind, eine oder mehrere weitere Befestigungsöffnungen (28, 30, 128, 130), die in dem proximalen Knochenplattenabschnitt (12, 112) und/oder dem distalen Knochenplattenabschnitt (14, 114) unterhalb der geschlitzten Positionierungsöffnung (22, 122) näher an dem distalen Längsende (20) gelegen sind als die geschlitzte Positionierungsöffnung (22, 122).

7. Knochenplatte (10, 110) nach Anspruch 6, wobei die eine oder die mehreren weiteren Befestigungsöffnungen (28, 30, 128, 130), die unterhalb der geschlitzten Positionierungsöffnung (22, 122) gelegen sind, gelegen sind
- zwischen der geschlitzten Positionierungsöffnung (22, 122) und der länglichen Öffnung (24, 124) im proximalen Knochenplattenabschnitt (12, 112) und, optional, dem distalen Knochenplattenabschnitt (14, 114), und, optional,
- unterhalb der länglichen Öffnung (24, 124) näher am distalen Längsende (20) als die längliche Öffnung (24, 124) im distalen Knochenplattenabschnitt (14, 114).

8. Knochenplatte (10, 110) nach einem der Ansprüche 4 bis 7, wobei
der distale Knochenplattenabschnitt (14, 114) keine Befestigungsöffnungen aufweist, die in der lateralen Richtung relativ zu der länglichen Öffnung (24, 124) und entlang der länglichen Öffnung (24, 124) gelegen sind.

9. Knochenplatte (10, 110) nach einem der Ansprüche 6 bis 8, wobei zumindest einige der weiteren Befestigungsöffnungen (28, 30, 64, 128, 130, 164) zusätzlich zu den beiden weiteren Befestigungsöffnungen, die unmittelbar benachbart und unterhalb der geschlitzten Positionierungsöffnung (28, 30, 128, 130) gelegen sind, Verriegelungsöffnungen sind.

10. Knochenplatte (10, 110) nach einem der vorhergehenden Ansprüche, wobei der proximale Knochenplattenabschnitt (12, 112) aufweist
- eine oder mehrere K-Draht-Öffnungen (32,66,132, 166), die dazu eingerichtet sind, einen K-Draht (48) aufzunehmen und oberhalb der geschlitzten Positionierungsöffnung (22, 122) in dem proximalen Knochenplattenabschnitt (12, 112) näher an dem proximalen Längsende (18) als die geschlitzte Positionierungsöffnung (22, 122) gelegen sind, und/oder
- eine oder mehrere K-Draht-Öffnungen (32, 132), die dazu eingerichtet sind, einen K-Draht (48) aufzunehmen und unterhalb der geschlitzten Positionierungsöffnung (22, 122) näher an dem distalen Längsende (14) gelegen sind als die geschlitzte Positionierungsöffnung (22, 122).

11. Knochenplatte (10, 110) nach Anspruch 10, wobei eine (66, 166) oder mehr der einen oder mehreren K-Draht-Öffnungen (32, 66, 132, 166), insbesondere eine (66, 166) der einen oder mehreren K-Draht-Öffnungen (32, 66, 132, 166), dazu eingerichtet sind, einen Stift (56) einer Zielvorrichtung (52) aufzunehmen, die dazu geeignet ist, die Befestigungselemente in die Knochenplatte einzuführen, um eine Drehung der Knochenplatte (10, 110) relativ zu der Zielvorrichtung (52) zu verhindern.

12. Knochenplatte (10, 110) nach einem der vorhergehenden Ansprüche, wobei
die Knochenplatte (10, 110) eine proximale Humerus-Knochenplatte ist,
der proximale Knochenplattenabschnitt (12, 112) dazu eingerichtet ist, an einem Humerus-Abschnitt in einem Bereich einer großen Tuberositas anzuliegen, und
der distale Knochenplattenabschnitt (14, 114) dazu eingerichtet ist, an einem Humerus-Schaftabschnitt anzuliegen.

13. Knochenplatte (10, 110) nach Anspruch 12, wobei die geschlitzte Positionierungsöffnung (22, 122) nahe einem Calcar-Bereich (74) des Humerus (40) gelegen ist, wenn die proximale Humerus-Knochenplatte an einem Humerus-Knochen befestigt ist.

14. Knochenplatte (10, 110) nach einem der vorhergehenden Ansprüche, wobei der proximale Knochenplattenabschnitt (12, 112) und der distale Knochenplattenabschnitt (14, 114) aneinander angrenzen.

## Revendications

1. Plaque vissée (10, 110) destinée à fixer des fragments osseux (46) à un os (40), comprenant
une partie proximale (12, 112) de la plaque vissée comprenant une extrémité longitudinale proximale (18) de la plaque vissée (10, 110) et une partie distale (14) de la plaque vissée comprenant une extrémité longitudinale distale (20) de la plaque vissée (10, 110), la plaque vissée (10, 110) s'étendant de l'extrémité longitudinale proximale (18) à l'extrémité longitudinale distale (20) dans une direction longitudinale, et la partie proximale (12, 112) de la plaque vissée présentant une largeur qui est supérieure à la largeur de la partie distale (14, 114) de la plaque vissée,
la partie proximale (12, 112) de la plaque vissée étant conçue pour venir en butée contre un fragment osseux (46) et la partie distale (14, 114) de la plaque vissée étant conçue pour venir en butée contre l'os (40),
la partie proximale (12, 112) de la plaque vissée comprenant une ouverture fendue de positionnement (22, 122) dont la longueur est parallèle à la direction longitudinale de la plaque vissée (10, 110) et configurée pour recevoir un élément de positionnement rigide allongé (48) et deux ouvertures de fixation (26, 126) conçues chacune pour recevoir une vis de fixation respective (72) destinée à fixer la plaque vissée (10, 110) à au moins l'un parmi l'os (40) et le fragment osseux (46),
dans laquelle la longueur de l'ouverture oblongue de positionnement (22, 122) dans la direction longitudinale de la plaque vissée (10, 110) est supérieure à la largeur de ladite ouverture oblongue de positionnement (22, 122) dans une direction latérale perpendiculaire à la direction longitudinale,
dans laquelle la largeur de l'ouverture oblongue de positionnement (22, 122) correspond sensiblement à la largeur de l'élément de positionnement rigide allongé (48) et l'ouverture oblongue de positionnement (22, 122) est conçue pour permettre à l'élément de positionnement rigide allongé (48) de coulisser le long et à l'intérieur de l'ouverture oblongue de positionnement (22, 122),
**caractérisée en ce que**
les deux ouvertures de fixation (26, 126) sont situées dans le sens latéral par rapport à l'ouverture oblongue de positionnement (22, 122), de part et d'autre de l'ouverture oblongue de positionnement (22, 122),
dans laquelle les deux ouvertures de fixation (26, 126) comportent une structure de verrouillage conçue pour verrouiller l'élément de fixation correspondant selon un angle choisi par rapport à la plaque vissée (10, 110), et
la plaque vissée (10, 110) comprenant deux autres ouvertures de fixation (28, 30, 128, 130) positionnées directement à côté et en dessous de l'ouverture oblongue de positionnement (22, 122), plus près de l'extrémité longitudinale distale (20) que l'ouverture oblongue de positionnement (22, 122) dans la partie proximale (12, 112) de la plaque vissée, les deux ouvertures de fixation supplémentaires (28, 30, 128, 130) comportant une structure de verrouillage conçue pour verrouiller l'élément de fixation respectif selon un angle sélectionné par rapport à la plaque vissée (10, 110).

2. Plaque vissée (400, 10, 110) selon l'une quelconque revendication 1, dans laquelle
la largeur maximale de l'ouverture oblongue de positionnement (22, 122) est comprise entre environ 1,0 et 3,0 mm.

3. Plaque vissée (110) selon la revendication 1 ou 2, dans laquelle
l'ouverture oblongue de positionnement (22, 122) est conçue pour recevoir une broche de Kirschner (48) en tant qu'élément de positionnement rigide allongé.

4. Plaque vissée (400, 10, 110) selon l'une quelconque des revendications précédentes, dans laquelle
la partie distale (14, 114) de la plaque vissée comprend une ouverture oblongue (24, 124) conçue pour recevoir un élément de fixation (72) et s'étendant dans le sens longitudinal de la plaque vissée (10, 110).

5. Plaque vissée (110) selon la revendication 4, dans laquelle
la largeur de l'ouverture oblongue de positionnement (22, 122) est inférieure à la largeur de l'ouverture oblongue (24, 124).

6. Plaque vissée (400, 10, 110) selon l'une quelconque des revendications précédentes, dans laquelle
la plaque vissée (10, 110)comprend en outre au moins l'une parmi
- une ou plusieurs ouvertures de fixation supplémentaires (28, 64, 128, 164) situées au-dessus de l'ouverture oblongue de positionnement (22, 122) dans la partie proximale (12, 112) de la plaque vissée, plus près de l'extrémité longitudinale proximale (18) que l'ouverture oblongue de positionnement (22, 122), et
- outre les deux ouvertures de fixation supplémentaires (28, 30, 128, 130) situées directement à côté et en dessous de l'ouverture oblongue de positionnement (22, 122), une ou plusieurs autres ouvertures de fixation (28, 30, 128, 130) situées en dessous de l'ouverture oblongue de positionnement (22, 122), plus près de l'extrémité longitudinale distale (20) que l'ouverture oblongue de positionnement (22, 122), dans au moins l'une parmi la partie proximale (12, 112) de la plaque vissée et la partie distale (14, 114) de la plaque vissée.

7. Plaque vissée (110) selon la revendication 6, dans laquelle
la ou les autres ouvertures de fixation (28, 30, 128, 130) situées en dessous de l'ouverture oblongue de positionnement (22, 122) sont disposées
- entre l'ouverture oblongue de positionnement (22, 122) et l'ouverture oblongue (24, 124) situées dans la partie proximale (12, 112) de la plaque vissée et, éventuellement, dans la partie distale (14, 114) de la plaque vissée, et, éventuellement,
- en dessous de l'ouverture oblongue (24, 124), plus près de l'extrémité longitudinale distale (20) que l'ouverture oblongue (24, 124) située dans la partie distale (14, 114) de la plaque vissée.

8. Plaque vissée (400, 10, 110) selon l'une quelconque des revendications 4 à 7, dans laquelle
la partie distale (14, 114) de la plaque vissée ne comporte aucune ouverture de fixation située dans la direction latérale par rapport à l'ouverture oblongue (24, 124) ni le long de l'ouverture oblongue (24, 124).

9. Plaque vissée (400, 10, 110) selon l'une quelconque des revendications 6 à 8, dans laquelle
au moins certaines des ouvertures de fixation supplémentaires (28, 30, 64, 128, 130, 164), situées en plus des deux ouvertures de fixation supplémentaires positionnées directement à côté et en dessous de l'ouverture oblongue de positionnement (28, 30, 128, 130), sont des ouvertures de verrouillage.

10. Plaque vissée (400, 10, 110) selon l'une quelconque des revendications précédentes, dans laquelle
la partie proximale (12, 112) de la plaque vissée comprend au moins l'une parmi
- une ou plusieurs ouvertures (32, 66, 132, 166) pour la broche de Kirschner conçues pour recevoir une broche de Kirschner (48) et positionnées au-dessus de l'ouverture oblongue de positionnement (22, 122) dans la partie proximale (12, 112) de la plaque vissée, plus près de l'extrémité longitudinale proximale (18) que l'ouverture oblongue de positionnement (22, 122), et
- une ou plusieurs ouvertures (32, 132) pour la broche de Kirschner conçues pour recevoir une broche de Kirschnerl (48) et situées en dessous de l'ouverture oblongue de positionnement (22, 122), plus près de l'extrémité longitudinale distale (14) que l'ouverture oblongue de positionnement (22, 122).

11. Plaque vissée (110) selon la revendication 10, dans laquelle
l'une (66, 166) ou plusieurs de la ou des ouvertures (32, 66, 132, 166) pour broches de Kirschner, en particulier l'une (66, 166) parmi la ou les ouvertures (32, 66, 132, 166) pour broches de Kirschner sont conçues pour recevoir une tige (56) d'un dispositif de visée (52) adapté pour insérer les éléments de fixation dans la plaque vissée afin d'empêcher la plaque vissée (10, 110) de tourner par rapport au dispositif de visée (52).

12. Plaque vissée (400, 10, 110) selon l'une quelconque des revendications précédentes,
la plaque vissée (10, 110) étant une plaque vissée pour l'humérus proximal,
la partie proximale (12, 112) de la plaque vissée est conçue pour venir en butée contre une partie de l'humérus située dans la région du tubercule majeur, et
la partie distale (14, 114) de la plaque vissée est conçue pour venir en butée contre une partie de la diaphyse humérale.

13. Plaque vissée (110) selon la revendication 12, dans laquelle
l'ouverture oblongue de positionnement (22, 122) est placée de manière à se trouver à proximité d'une région du calcar (74) de l'humérus (40), lorsque la plaque vissée humérale proximale est fixée à l'humérus.

14. Plaque vissée (10, 110) selon l'une quelconque des revendications précédentes, dans laquelle
la partie proximale (12, 112) de la plaque vissée et la partie distale (14, 114) de la plaque vissée sont contiguës l'une à l'autre.
